# DEMANDE DE BREVET EUROPEEN

(11) **EP 4 035 592 A1**
(43) Date de publication de la demande: **03.08.2022**
(21) Numéro de dépôt: 22154059.4
(22) Date de dépôt: 28.01.2022
(51) Int. Cl.: A61B 5/00, H04M 1/02, H04M 1/03, H04M 1/05, H04R 1/14, H04W 4/30, H04W 4/90, H04M 3/51, H04L 67/00, H04R 5/033, H04W 88/02, A61B 5/0205

(54) **SYSTÈME COGNITIF INTELLIGENT DE PILOTAGE ET D'ASSISTANCE AUX OPÉRATEURS DISTANTS EN INTERVENTIONS TECHNIQUES SENSIBLES**

(30) Priorité: 29.01.2021 FR 2100878
(71) Demandeur: Finesenses, 75008 Paris (FR)
(72) Inventeur: PETROS, Olivier, 75008 PARIS (FR); DELAVAUD, Christian, 33200 BORDEAUX (FR)
(74) Mandataire: Ipside

(57) **Abrégé**

L'invention concerne un système de support et contrôle (30) de missions d'intervention, comprenant :
a. Un système multimédia (31), équipant un opérateur, comportant :
i. Un dispositif de communication (310),
ii. Un système audio (311) ;
iii. Un système vidéo (312) ;

b. Un système de contrôle (32) comprenant une mémoire (320) stockant une séquence d'instructions prédéterminées et des moyens de traitement (321);
caractérisé en ce que le système de contrôle (32) est agencé pour :
i. transmettre (42) chacune des instructions au dispositif de communication (310), agencé pour relayer cette instruction au système audio (311) et/ou vidéo (312) pour être transmise à l'opérateur ;
ii. recevoir (45) du dispositif de communication (310), un signal audio (43) et/ou vidéo (44) acquis par le système audio (311) et/ou vidéo (312) ;
iii. traiter (46) le signal par les moyens de traitement (321) et valider (47), l'exécution de chaque instruction (42).

## Description

### Domaine technique.

L'invention a pour objet un système de support et contrôle de missions d'intervention techniques.

L'invention se rapporte au domaine technique des systèmes de communication et notamment aux systèmes de communication à plus ou moins longue portée.

### Etat de la technique.

Dans de nombreux domaines, des intervenants effectuent des opérations nécessitant une importante précision d'exécution des gestes et une traçabilité des étapes de réalisation de l'opération afin de sécuriser celle-ci. Du fait de la rigueur imposée par de telles opérations, celles-ci sont généralement encadrées par une personne référente en charge de piloter et de contrôler l'intervenant. Cet encadrement permet de suivre les diverses étapes de l'opération et de sécuriser celles-ci en apportant un soutien et/ou des corrections au fur et à mesure de la réalisation de l'opération par l'intervenant.

Ces interventions techniques peuvent être des opérations de maintenance ou de surveillance et sont souvent réalisées dans des environnements complexes dotés de contraintes physiques ou réglementaires fortes, dans lesquels la sécurité des intervenants est primordiale. Afin d'assurer la sécurité des intervenants, ceux-ci ont pour habitude de porter des équipements à la fois de protection physique et de communication.

Il est ainsi connu d'utiliser des objets frontaux pour la visualisation comme des caméras équipées de micros fixées sur la poitrine ou sur la tête de l'intervenant, par exemples des lunettes, permettant à l'encadrant à distance de visualiser les gestes effectués. Cette utilisation de caméra n'est pas optimale puisque l'utilisation sur des lunettes n'est pas adaptée à la vue de l'intervenant tandis que le positionnement sur la poitrine ne permet pas à l'encadrant de suivre le regard de l'intervenant. De plus ces outils ne sont pas optimaux lorsque les intervenants sont dans des milieux hostiles puisqu'ils ne permettent pas le suivi physiologique de l'intervenant. Afin de répondre à ce besoin, il serait alors nécessaire d'ajouter une pluralité de capteurs sur le corps de l'intervenant. Plus le nombre d'éléments à porter par l'intervenant est élevé et plus il y aura d'informations à transmettre à l'encadrant, rendant le dispositif peut usuel et encombrant, notamment lorsque l'intervenant doit porter des équipements de type combinaisons, gants, etc.

De plus, ce type d'outils ne permettent pas de valider les étapes de réalisation de la mission. Il est donc nécessaire avant l'intervention de réaliser des réunions préliminaires dans lesquelles les étapes de réalisation de la mission doivent être validées, répétées et enregistrées par l'opérateur. Selon le type d'intervention ou de mission et sa dangerosité, il peut être difficile pour l'opérateur de retenir l'ensemble des étapes. Le stress aidant, l'opérateur peut voir sa mémoire défaillir et ainsi ne pas enchainer les étapes de réalisation dans le bon ordre.

Pour le secteur industriel, il existe ainsi un besoin pour un système ergonomique, autonome en énergie pour permettre l'exécution d'opérations d'intervention de façon rigoureuse, dont la communication avec l'encadrant est continue tout en permettant de suivre physiquement et physiologiquement l'état de l'intervenant.

Dans le domaine de la sécurité de forces organisées, il existe un besoin pour une solution permettant la communication entre des agents de terrain et un responsable d'opérations, tout en facilitant l'obtention de preuves audio et vidéo par les agents, ceci à des fins de traçage des opérations, de coordination et de contrôle des agents de terrain ou d'enquête. L'utilisation de la vidéo permet d'offrir une double vigilance et de rassurer à la fois les agents de terrain et les superviseurs. De façon similaire au domaine de la surveillance ou de la maintenance d'un site industriel, ce besoin requiert que la solution soit ergonomique, autonome en énergie, permettant une communication continue avec le responsable d'opérations tout en permettant de suivre physiquement et physiologiquement son état.

La présente invention se place dans ces différents contextes et visent à répondre à tout ou partie de ces besoins.

### Présentation de l'invention.

L'invention a pour objet un système de support et contrôle de missions d'intervention techniques, comprenant
- un système multimédia, destiné à équiper un opérateur, et comportant un dispositif de communication pour la transmission et la réception de données, un système d'acquisition et de transmission audio, un système d'acquisition et de transmission vidéo ;
- un système de contrôle centralisé et distant du système multimédia, comprenant une mémoire dans laquelle est stockée au moins une séquence d'instructions audio et/ou vidéo prédéterminées et un moyen de traitement de signaux audio et/ou vidéo ;

Le système est remarquable en ce que le système de contrôle est agencé pour
- transmettre de façon séquentielle chacune des instructions de la séquence d'instructions au dispositif de communication du système multimédia, lequel est agencé pour relayer cette instruction au système d'acquisition et de transmission audio et/ou vidéo pour être transmise à l'opérateur ;
- recevoir du dispositif de communication de l'équipement multimédia, en réponse à chaque transmission d'une instruction, un signal audio et/ou vidéo acquis par le système d'acquisition et de transmission audio et/ou vidéo du système multimédia ;
- traiter le signal audio et/ou vidéo reçu par le moyen de traitement de signaux audio et/ou vidéo et valider, en fonction de ce traitement, l'exécution par l'opérateur de chaque instruction transmise, la transmission d'au moins une instruction de la séquence d'instructions audio étant conditionnée à la validation de l'exécution de l'instruction précédente de la séquence d'instructions.

Le système peut servir de support et de contrôle à des missions d'intervention humaines comme par exemple dans des conditions d'utilisation sur un site industriel ou dans des manœuvre de sécurité. L'usage industriel peut par exemple être lors de maraudes ou d'opérations de maintenance dans des sites sensibles ou à risques comme des sites chimiques, nucléaires, des environnements contenant de l'amiante, des cuveries ou des cales de navire. Les manœuvres de sécurité peuvent par exemple être lors d'interventions délicates de missions dans lesquelles la traçabilité et les preuves sont importantes comme par exemple le contrôle et la coordination d'interventions, les supports d'enquête, la formation de groupe. Ce type d'utilisation peut notamment permettre un contrôle étape par étape de manière scriptée de l'opération à réaliser par l'utilisateur.

Le système multimédia peut se présenter sous diverses formes, comme par exemple un équipement destiné à être porté par l'opérateur comme une sangle à porter sur la poitrine, un casque, un bandeau de tête, un tour de cou, un manchon à porter sur le bras, un collier, une paire de lunettes, ou encore comme un équipement destiné à être saisi et manipulé par l'opérateur, comme une tablette tactile ou un téléphone intelligent. Eventuellement, le système multimédia peut être composé de différents équipements pouvant être portés par l'opérateur en différents endroits. Le système multimédia doit pouvoir être porté sans gêner les mouvements de l'opérateur pendant qu'il réalise les étapes de sa mission.

Le système multimédia comporte notamment une batterie. La batterie est, par exemple, de type lithium titanate. La batterie peut par exemple être clipsée ou aimantée sur le système multimédia. Le fait que le système multimédia utilise une batterie permet au système de support et de contrôle d'être autonome pendant une période d'au moins plusieurs heures permettant à l'opérateur de réaliser la mission d'intervention technique.

L'utilisation du système d'acquisition audio peut permettre une traçabilité de l'opération réalisée, par exemple par enregistrement des dires de l'utilisateur. L'exploitation des données audio du système d'acquisition audio peut permettre de former et de sensibiliser des utilisateurs aux opérations réalisées.

L'utilisation du système d'acquisition vidéo peut permettre une traçabilité de l'opération réalisée, par exemple par enregistrement des faits et gestes de l'utilisateur. L'exploitation des données vidéo du système d'acquisition vidéo peut permettre de former et de sensibiliser des utilisateurs aux opérations réalisées. Dès l'activation du système d'acquisition vidéo, les images peuvent être enregistrées en continu jusqu'à la fin de la procédure et l'extinction dudit système. Le système d'acquisition vidéo peut également comporter un système d'éclairage, par exemple infra-rouge, notamment permettant l'acquisition d'images y compris lorsque l'utilisateur est dans un environnement peu ou non éclairé, par exemple lors d'une intervention de nuit L'enregistrement de la séquence vidéo peut servir à des fins de contrôles pendant les opérations mais également à des fins d'expertises en cas de problèmes suite à l'intervention.

Le dispositif de communication peut être distant du système multimédia, c'est-à-dire que ledit système multimédia et ledit dispositif de communication sont deux éléments physiquement distincts. Avantageusement, le dispositif de communication distant peut être un dispositif média supplémentaire porté par l'opérateur, par exemple de type tablette ou téléphone intelligent. Un relai de communication peut donc être mis en place entre le dispositif média supplémentaire et système d'acquisition audio et vidéo du système multimédia. C'est le dispositif de communication qui permet de transmettre les données multimédia et/ou physiologiques entre l'opérateur et le serveur distant.

Le système de contrôle peut comprendre une mémoire permettant de stocker au moins une séquence d'instructions audio et/ou vidéo prédéterminées. Le système de contrôle permet, dans un mode de réalisation, de stocker une pluralité de séquences d'instructions prédéterminées. Le système peut comprendre un moyen de traitement des signaux audio et/ou vidéo transmis et reçus. Lors de la mise sous tension du système multimédia, le système de contrôle peut mettre en œuvre un test de vérification du système d'acquisition et de transmission audio, le test de vérification peut également être réalisé pour le système d'acquisition et de transmission vidéo, pour le système de communication, pour le système de localisation et pour la batterie quand le système comporte l'une de ces fonctions. Si le test est validé par le système de contrôle, et que chaque composant est fonctionnel, ledit système de contrôle autorise l'exploitation du système. La validation du test par le système de contrôle est l'étape préliminaire à la validation future des étapes de réalisation de l'opération par l'opérateur.

Dans un exemple de réalisation, le système de contrôle réalise le test de vérification par émission d'un signal audio et/ou vidéo périodique par l'émetteur, la réception d'un signal audio et/ou vidéo périodique par le récepteur, le calcul d'un déphasage entre les signaux audio et/ou vidéo émis et reçu, le test étant considéré comme réussi si le déphasage reste constant et inférieur à un seuil donné pendant toute la réception du signal audio et/ou vidéo.

Avantageusement, chaque instruction de ladite séquence d'instructions est un signal audio et/ou vidéo. Le cas échéant, chacun de ces signaux audio et/ou vidéo peut être émis par un utilisateur référent humain ou peut être généré de manière automatique, par exemple à l'aide d'une méthode de synthèse vocale à partir d'une instruction écrite.

Le système de contrôle peut recevoir un signal audio et/ou vidéo acquis par le(s) système(s) d'acquisition et de transmission audio et/ou vidéo du système multimédia, en réponse à la transmission d'une instruction de la séquence prédéterminée. Avantageusement, le signal audio et/ou vidéo acquis provient de l'opérateur qui peut par exemple énoncer les actes qu'il est en train de réaliser ou par exemple filmer ses gestes. Par exemple les mots énoncés par l'opérateurs peuvent être analysés par le système de contrôle qui peut le traduire, notamment automatiquement, en séquence de texte écrit.

Avantageusement, les moyens de traitement de signaux audio et/ou vidéo du système de contrôle peuvent être agencés pour traiter le signal audio et/ou vidéo acquis et reçu de sorte à valider ou invalider l'exécution de l'instruction précédemment transmise à l'opérateur. La validation de l'exécution de l'instruction précédemment transmise pourra par exemple être mise en œuvre par les moyens de traitement de signaux audio et/ou vidéo par comparaison du contenu du signal audio et/ou vidéo acquis et reçu à une consigne audio et/ou vidéo prédéterminée et associée à ladite instruction précédemment transmise. Le cas échéant, le système de contrôle transmet l'instruction suivante seulement si l'étape précédente a été validée par acquittement audio et/ou validation vidéo de la ou de chaque étape. Si on le souhaite, ce traitement de signal audio et/ou vidéo pourra être mis en œuvre pour chaque signal audio et/ou vidéo reçu du système multimédia en réponse à la transmission de chaque instruction ou en variante pour seulement certains de ces signaux audio et/ou vidéo. Le cas échéant, certaines seulement des transmissions d'instruction pourront être conditionnées à la validation de l'exécution de l'instruction précédente. En variante, chaque transmission d'instruction sera conditionnée à la validation de l'exécution de l'instruction précédente.

Dans un exemple de réalisation, le dispositif de communication permet la communication entre l'opérateur et au moins un utilisateur distant. On entend par utilisateur distant, un utilisateur qui se trouve éloigné de l'utilisateur porteur et dont la distance les séparant ne permet pas une interaction orale directe entre eux. Ils peuvent donc par exemple être éloignés l'un de l'autre d'une distance d'au moins 10 m, ou par exemple être dans deux pièces distinctes, ou par exemple être à plusieurs kilomètres l'un de l'autre voire dans deux villes distinctes ou dans deux pays distincts. Avantageusement, par utilisateur distant on entend notamment une personne unique, une pluralité de personnes regroupées dans un même endroit, ou encore une pluralité de personnes dans une pluralité d'endroits. Le système d'acquisition et de transmission audio permet par exemple à l'utilisateur et à l'utilisateur distant de communiquer oralement entre eux.

Avantageusement, les moyens de traitement de signaux audio et/ou vidéo du système de contrôle comprennent un moyen de transcription vocale apte à transcrire un signal vocal en texte, et le système de contrôle est agencé, lors de la réception du dispositif de communication de l'équipement multimédia d'un signal vocal acquis par le système d'acquisition et de transmission audio, pour transcrire ce signal vocal en texte et stocker ce texte dans ladite mémoire du système de contrôle. La transcription sous format texte du signal vocal permet de faciliter la relecture et le contrôle ultérieur des étapes de l'opération qui ont été réalisées par l'opérateur. Le cas échéant, la validation de l'exécution de l'instruction précédemment transmise pourra par exemple être mise en œuvre par les moyens de traitement de signaux audio et/ou vidéo par comparaison du texte transcrit à partir du signal vocal acquis et reçu à une consigne écrite prédéterminée et associée à ladite instruction précédemment transmise.

Le moyen de transcription vocal comprend avantageusement un moyen de reconnaissance vocale apte à identifier l'identité de la personne ayant émis ledit signal vocal. De la sorte, il est possible de distinguer si c'est bien l'opérateur qui parle ou si c'est une tierce personne.

Avantageusement, une pluralité de séquence d'instructions audio et/ou vidéo prédéterminées sont stockées dans la mémoire du système de contrôle en étant chacune associée à un identifiant donné d'une intervention technique à exécuter par un opérateur, et le système de contrôle comporte une interface de sélection d'un identifiant d'une intervention technique à exécuter par un opérateur parmi une pluralité d'identifiants.

En réponse à cette sélection, le système de contrôle est agencé pour transmettre séquentiellement la ou les instructions de la séquence prédéterminée au dispositif de communication du système multimédia, lequel est agencé pour relayer la ou chaque instruction de la séquence au(x) système(s) d'acquisition et de transmission audio et/ou vidéo, de sorte que l'opérateur le reçoive.

Si on le souhaite, le système de contrôle comporte une interface de saisie d'une séquence d'instructions audio et/ou vidéo prédéterminées, associée à une intervention donnée, chaque instruction décrivant une étape de ladite intervention en identifiant au moins l'un ou plusieurs des paramètres d'étapes suivants : au moins un intervenant à l'étape, au moins une localisation de l'étape, au moins un outil nécessaire l'étape, au moins une pièce à manipuler dans l'étape, au moins un mode opératoire de l'étape.

Par exemple, la au moins une pièce à manipuler dans l'étape peut être solidaire de l'infrastructure ou externe à l'infrastructure, comme par exemple une manivelle, une poignée, un levier, un interrupteur de l'infrastructure ou un composant destiné à être installé dans l'infrastructure, notamment en vue du remplacement d'un autre composant.

Avantageusement, le système multimédia comporte au moins un capteur apte à mesurer un paramètre de l'environnement de l'opérateur ou un paramètre physiologique de l'opérateur, le dispositif de communication étant agencé pour transmettre au système de contrôle des données relatives audit paramètre mesuré par le capteur, et le système de contrôle est agencé pour comparer lesdites données reçues à une valeur seuil prédéterminée et pour, en fonction de cette comparaison, émettre un signal d'alerte audio et/ou vidéo au dispositif de communication du système multimédia, lequel est agencé pour relayer ce signal d'alerte au système d'acquisition et de transmission audio et/ou vidéo pour être transmis à l'opérateur. De préférence, le système multimédia peut comporter une pluralité de capteurs et un calculateur, par exemple montés sur une même carte électronique. Avantageusement, le ou chaque capteur est apte à mesurer un paramètre particulier de l'environnement de l'opérateur ou un paramètre physiologique de l'opérateur. Le système multimédia peut notamment comporter un capteur de pression, un capteur d'hygrométrie, un capteur de température, un capteur du niveau de CO₂ et de O₂, un altimètre, un radiamètre ou un contaminamètre, ou encore un accéléromètre. Le capteur du niveau de CO₂ et de O₂ permet d'estimer la dangerosité aérobie du milieu environnant. Un milieu aérobie est un milieu contenant du CO₂ et donc dangereux pour l'utilisateur. Le radiamètre permet de mesurer les radiations dans l'environnement de l'utilisateur, permettant de détecter et de quantifier les rayonnements X, béta et gamma. Le contaminamètre permet de mesurer une contamination radioactive sur une surface ou dans un environnement donné. L'accéléromètre permet de suivre les accélérations de l'utilisateur et donc de détecter une chute, un malaise ou des difficultés d'ascension.

Le système de contrôle centralisé peut fixer les seuils admissibles pour les divers paramètres mesurés et ainsi fixer la graduation de la situation. En fonctions des seuils déterminés, des dispositifs d'alerte peuvent se déclencher et ainsi prévenir l'opérateur et le système de contrôle centralisé distant des potentiels dangers auxquels l'utilisateur est exposé.

Le calculateur du système multimédia permet de mesurer et d'étudier les données physiologiques telles que le rythme cardiaque et le rythme respiratoire mesurés grâce au laryngophone du système d'acquisition audio. La transmission des données enregistrées par le ou chaque capteur est transmise au système de contrôle périodiquement, par exemple en temps réel ou à des intervalles de temps réguliers, tout au long de la mission.

Avantageusement, le système multimédia comporte au moins un système de localisation de l'opérateur, le dispositif de communication étant agencé pour transmettre au système de contrôle des données relatives à la position de l'opérateur estimé par le système de localisation.

Dans un exemple de réalisation, le système de localisation peut comporter une puce de localisation GPS (Global Positioning System, en français : « Système mondial de positionnement »). Dans cet exemple, il est ainsi possible de localiser l'opérateur lorsque celui-ci se déplace en dehors d'une infrastructure. Dans un exemple de réalisation alternatif ou cumulatif, le dispositif de communication pour la transmission et la réception de données comporte un récepteur, et le système de support et de contrôle comporte une pluralité d'émetteurs distribués dans un bâtiment au sein duquel doit se dérouler une intervention, et le système de localisation est agencé pour mettre en œuvre un procédé de biangulation/triangulation des signaux émis par les émetteurs et reçus par le récepteur. Dans cet exemple, il est possible de localiser l'opérateur lorsqu'il se déplace dans une infrastructure. Si on le souhaite, le système de localisation peut comporter un altimètre, le système de localisation étant agencé pour estimer une position de l'opérateur selon trois axes. Le système de localisation est important, en particulier lorsque l'opérateur se retrouve dans un milieu isolé. Ce système permet la localisation précise de l'opérateur, sa vitesse de déplacement et permet également d'enregistrer son parcours. En fonction du chemin que doit parcourir l'opérateur, par exemple, montée de marches et d'une échelle, la localisation peut permettre de déterminer si une situation est normale ou anormale, par exemple en cas de chute ou de malaise. Dans un autre exemple d'utilisation, un opérateur devant se déplacer sur un site de taille importante peut se perdre, la communication avec l'utilisateur distant peut ainsi permettre de guider l'utilisateur sur le bon itinéraire à parcourir.

Le système de contrôle du système multimédia est agencé pour comparer les données de position de l'opérateur à des données de position prédéterminées, par exemple associées aux instructions de la séquence d'instructions, la transmission d'une instruction de la séquence d'instructions audio étant conditionnée au succès de la comparaison des dernières données de position reçues aux données de position prédéterminées associée à l'instruction précédente de la séquence d'instructions.

Avantageusement, le système de contrôle est apte à identifier un début d'une intervention technique à exécuter par un opérateur et à identifier au moins un outil nécessaire à l'exécution de cette intervention, le système de contrôle est agencé, au préalable de la transmission de la première instruction de la séquence d'instructions associée à cette intervention, pour recevoir du dispositif de communication de l'équipement multimédia, un signal vidéo acquis par le système d'acquisition et de transmission vidéo du système multimédia et pour contrôler la présence sur ce signal vidéo, à l'aide du moyen de traitement de signaux audio et/ou vidéo, de chaque outil nécessaire préalablement identifié.

Dans ce mode de réalisation, la transmission d'une instruction de la séquence d'instructions audio est conditionnée à la présence desdits outils. Par exemple, chaque outil est muni d'un code en deux dimensions de type QR code (pour « quick response code », en français « code à réponse rapide »), d'un code barre ou d'une reconnaissance vidéo de sa forme. Le cas échéant, les moyens de traitement audio et/ou vidéo sont agencés pour mettre en œuvre un algorithme de lecture d'un QR code, d'un algorithme de lecture d'un code barre d'un algorithme de lecture d'une étiquette et/ou d'un algorithme de reconnaissance de forme dans une image, par exemple d'un contour d'un outil et/ou d'une inscription alphanumérique.

Avantageusement, pour au moins un signal audio et/ou vidéo reçu du dispositif de communication de l'équipement multimédia en réponse à la transmission d'une instruction, le système de contrôle est agencé pour :
a. si l'instruction transmise requiert l'utilisation d'un outil donné, contrôler la mention dans le signal audio reçu ou la présence sur le signal vidéo reçu, à l'aide du moyen de traitement de signaux audio et/ou vidéo, de cet outil donné ; et/ou
b. si l'instruction transmise requiert la manipulation d'une pièce donnée, contrôler la mention dans le signal audio reçu ou la présence sur le signal vidéo reçu, à l'aide du moyen de traitement de signaux audio et/ou vidéo, de cette pièce donnée ; et/ou
c. si l'instruction transmise requiert un mode opératoire donné, contrôler la mention dans le signal audio reçu ou la réalisation sur le signal vidéo reçu, à l'aide du moyen de traitement de signaux audio et/ou vidéo, de ce mode opératoire donné.

Si le système de contrôle valide l'exécution d'une instruction, le système de contrôle est agencé pour transmettre, au préalable de la transmission de l'instruction suivante, un signal audio et/ou vidéo de confirmation de la bonne exécution de l'instruction à destination de l'opérateur.

Avantageusement, l'équipement multimédia est un collier autonome, destiné à être porté par l'opérateur, et intégrant le dispositif de communication et les systèmes d'acquisition et de transmission audio et vidéo.

Dans un exemple de réalisation, le collier autonome, destiné à être porté par l'opérateur, est agencé par exemple pour être disposé autour du cou de l'utilisateur lui permettant ainsi d'être libre de ses mouvements. Le collier autonome peut comporter un dispositif de communication pour la transmission et la réception de données et un système d'acquisition et de transmission audio, le système de communication comprenant une unité de communication distante du collier autonome et apte à communiquer avec le dispositif de communication du collier pour lui transmettre un message audio, émis à destination de l'utilisateur par le système d'acquisition et de transmission du collier, et/ou pour en recevoir un message audio acquis par le système d'acquisition et de transmission audio du collier. De préférence, le dispositif de communication est un dispositif de communication sans-fil apte à échanger des données avec l'unité de communication selon un protocole donné, par exemple un protocole WiFi, un protocole LTE de 4^{ème} génération et/ou un protocole LTE de 5^{ème} génération.

Avantageusement, le haut-parleur est un transducteur ostéophonique et un laryngophone. Le transducteur ostéophonique est un type de haut-parleur par conduction osseuse permettant de transmettre une fréquence d'un signal sonore à l'utilisateur en éliminant plus de 80% des bruits environnants. En effet, la conduction osseuse permet la propagation du son jusqu'à l'oreille interne par les os du crâne et/ou de la mâchoire. Ainsi, un utilisateur étant exposé à un environnement fortement bruyant et portant des bouchons de protection d'oreilles peut tout de même entendre les messages audio qui lui sont transmis par l'utilisateur distant. Dans le mode de réalisation où l'opérateur communique avec au moins un utilisateur distant, en fonction de l'état de l'opérateur, l'utilisateur distant peut prendre en charge la gestion du stress de l'utilisateur.

Le laryngophone permet de transmettre à l'opérateur, les instructions données par le système de contrôle centralisé ou les propos de l'utilisateur distant. Le laryngophone est un type de microphone conçu pour être placé sur la gorge de l'utilisateur, en contact avec sa peau, afin de capter les vibrations de son larynx et de ses cordes vocales, et ainsi restituer sa voix et ses paroles. C'est un type particulier de microphone de contact. En outre, ce laryngophone permet avantageusement de prendre le pouls et le rythme respiratoire de l'utilisateur permettant de déterminer le statut physiologique et psychologique de l'utilisateur de sorte que, en fonction de cet état, l'utilisateur distant puisse prendre en charge la gestion du stress de l'utilisateur.

Avantageusement, l'utilisation du transducteur ostéophonique et du laryngophone permettent une communication dans laquelle l'opérateur peut être entendu et peut entendre sans altération instructions orales qui lui sont données notamment dans des environnements sonores polluants. L'utilisation de ce type de communication audio permet à l'opérateur de porter des protections auditives tout en pouvant recevoir les instructions audio du système de contrôle.

Avantageusement, le collier comporte un système d'acquisition vidéo, et l'unité de communication distante du collier autonome est apte à recevoir, depuis le dispositif de communication du collier, un message vidéo acquis par le système d'acquisition vidéo du collier.

Avantageusement, le collier comporte un arceau destiné à venir autour du cou de l'utilisateur. L'arceau se présente sous la forme d'un arc de cercle flexible. L'arceau peut ainsi s'adapter au tour de cou de l'utilisateur. L'arceau peut être constitué d'un matériau flexible ou d'une combinaison de matériaux flexibles. L'arceau peut ainsi être installé autour du cou de l'utilisateur et être facilement adapté à diverses morphologies homme ou femme. La surface interne de l'arceau peut être recouverte d'un revêtement anti-irritation. Par exemple, ce revêtement peut se présenter sous la forme d'un coussin anti-irritation notamment sous la forme d'un coussin dermique. L'arceau peut comporter un bouton permettant la mise sous tension et l'extinction du collier, avantageusement disposé sur le côté de l'arceau. L'arceau peut comporter un voyant de fonctionnement apte à indiquer la mise sous tension du collier. Le voyant de fonctionnement peut permettre un suivi en temps réel du niveau de charge de la batterie.

Le dispositif de communication comporte une antenne intégrée dans ledit arceau et le système d'acquisition et/ou de transmission audio comporte un haut-parleur et un microphone intégrés dans ledit arceau du collier. Le collier comporte par exemple, une pluralité de capteurs intégrés dans ledit arceau, chacun apte à mesurer un paramètre de l'environnement de l'utilisateur. Le système d'acquisition audio et le cas échéant, le système d'acquisition vidéo, sont disposés au niveau d'une extrémité de l'arceau. Ainsi lorsque l'utilisateur porte l'arceau, le système d'acquisition vidéo est disposé avantageusement à l'avant du cou de l'utilisateur et le système d'acquisition audio est disposé à proximité des os de la mâchoire de l'utilisateur. Dans un autre mode de réalisation, le système d'acquisition audio est disposé à l'arrière du cou, à proximité des os de la tête de l'utilisateur, par exemple des os du crâne ou des os de la mâchoire. Le système d'acquisition vidéo peut être disposé de telle sorte que quelle que soit la morphologie de l'utilisateur, ledit système d'acquisition vidéo soit réglé pour un champ de capture idéal.

Les autres éléments constitutifs de l'arceau sont par exemple disposés sur le pourtour dudit arceau afin que l'utilisateur ne subisse pas une sensation de gêne pendant le port du collier. En particulier, un système de traitement de l'information, relié à l'antenne, peut être installé à l'arrière de l'arceau, avec la batterie et la carte électronique comportant des capteurs.

Avantageusement, le collier comporte un système de localisation de l'utilisateur. Par exemple, le système de localisation est intégré audit arceau.

Le collier comporte un capteur apte à mesurer un paramètre physiologique de l'utilisateur. De préférence, ledit capteur est intégré audit arceau. Dans un mode de réalisation de l'invention, le collier comporte un calculateur agencé pour mesurer un ou plusieurs paramètres physiologiques de l'utilisateur à partir des données audios acquises par le système d'acquisition et de transmission audio, lequel forme le capteur apte à mesurer un paramètre physiologique de l'utilisateur. Le collier peut par exemple comporter une unité de contrôle agencée pour détecter une mise sous tension du collier et pour mettre en œuvre, à la suite de cette détection, un procédé de test de synchronisation entre un émetteur et un récepteur du système d'acquisition et de transmission audio. Le cas échéant, l'unité de contrôle peut être intégrée audit arceau.

Avantageusement, l'utilisation d'un arceau regroupant l'ensemble des éléments permet à l'utilisateur de porter des équipements de protection individuels tels que des combinaisons ou des tenues ventilées pour le confinement en milieu nucléaire ou chimique, des casques ou encore des gants. L'utilisation d'un arceau permet de rassembler l'ensemble des éléments du collier en un seul élément.

Dans un mode de réalisation, le système comprend au moins deux systèmes multimédias, chacun destiné à équiper un opérateur distinct, et le système de contrôle est agencé pour transmettre de façon séquentielle chacune des instructions de ladite séquence d'instructions à l'un ou l'autre des dispositifs de communication des systèmes multimédia. Dans cet exemple, la mission est réalisée par une pluralité d'opérateurs portant chacun un système multimédia et est contrôlée par un unique système de contrôle centralisé.

L'invention a également pour objet un système multimédia d'un système de support et contrôle de missions d'intervention techniques, le système multimédia étant destiné à équiper un opérateur. Avantageusement, le système multimédia est un collier.

L'invention a également pour objet un procédé de support et contrôle de missions d'intervention techniques, caractérisé en ce qu'il est mis en œuvre par un système de support et contrôle de missions d'intervention techniques selon l'invention.

### Brève description des figures.

D'autres avantages et caractéristiques de la présente invention sont maintenant décrits à l'aide d'exemples uniquement illustratifs et nullement limitatifs de la portée de l'invention, et à partir des dessins annexés, dessins sur lesquels les différentes figures représentent :
[fig.1] représente schématiquement un système de support et de contrôle selon un premier mode de réalisation
[fig.2] représente schématiquement un système de support et de contrôle selon un deuxième mode de réalisation
[fig.3] représente schématiquement le fonctionnement d'un système de contrôle générant une séquence d'instructions.
[fig.4] représente schématiquement un champ de vue d'une caméra et un mode de fonctionnement de reconnaissance d'outil.

Dans la description qui suit, les éléments identiques, par structure ou par fonction, apparaissant sur différentes figures conservent, sauf précision contraire, les mêmes références.

### Description des modes de réalisation.

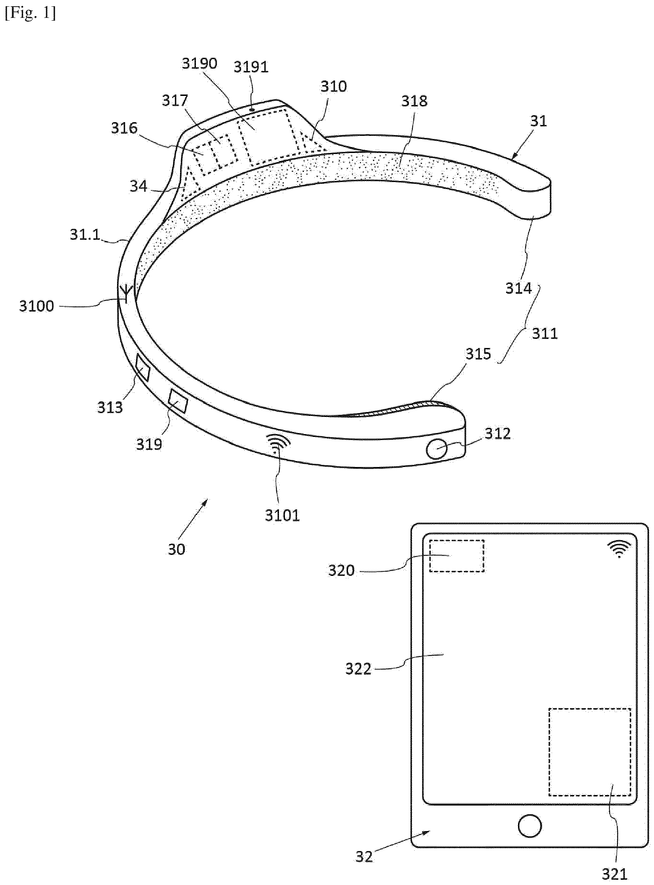

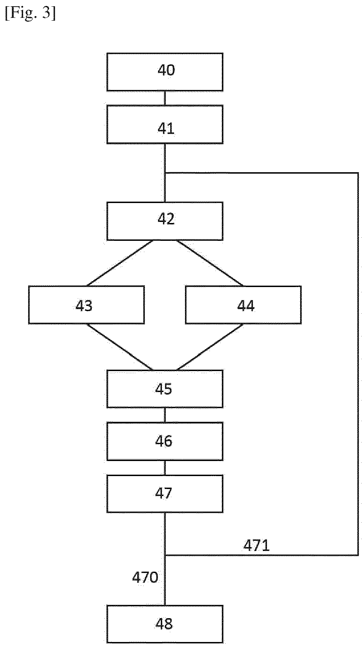

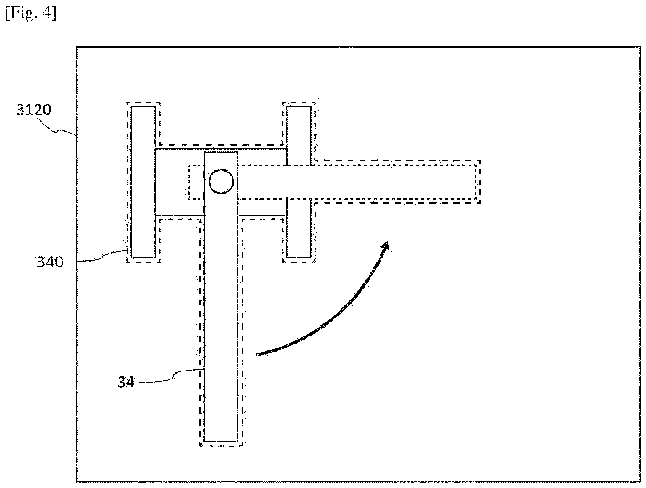

On a représenté en [fig.1] un système de support et de contrôle 30 selon un mode de réalisation de l'invention. Ce système 30 est décrit en lien avec les [fig.3] et [fig.4] qui illustrent chacune une partie ou la totalité du fonctionnement d'un système de contrôle 32 de ce système 30.

Le système de support et de contrôle 30 comprend un système multimédia 31 destiné à équiper un opérateur et un système de contrôle 32 centralisé et distant du système multimédia 31.

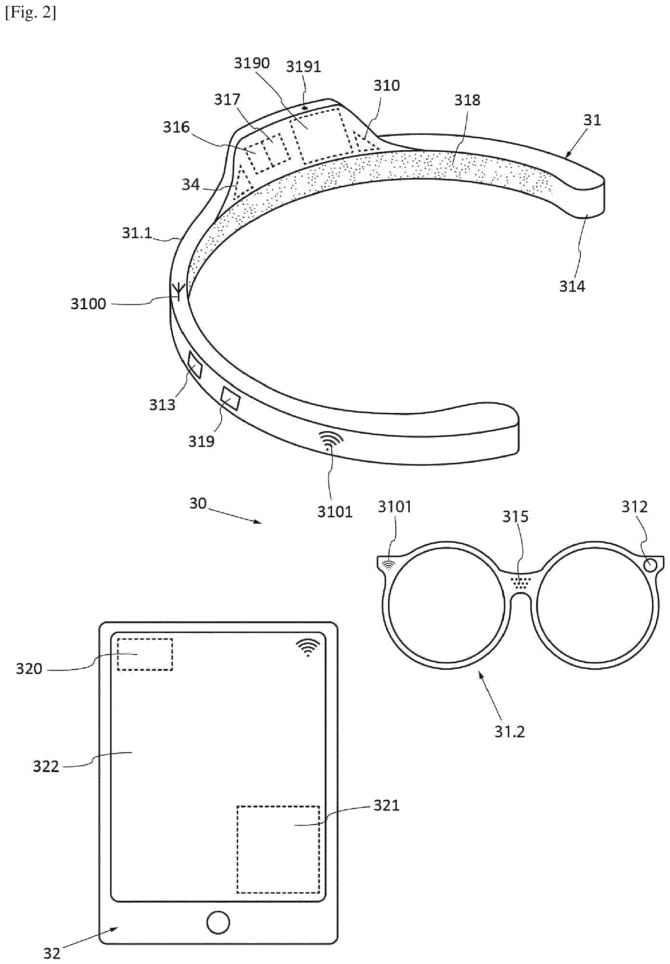

Dans les modes de réalisation décrits aux [fig.1] et [fig.2], le système multimédia 31 est un collier comportant un arceau 31.1 destiné à être porté autour du cou de l'opérateur. L'arceau 31.1 se présente sous la forme d'un arc de cercle formé en un matériau ou un ensemble de matériaux flexible(s). L'arceau 31.1 présente une face interne, face au cou de l'utilisateur et une face externe, opposée à la face interne. La surface interne de l'arceau 31.1 est recouverte d'un coussin dermique 318. Le coussin dermique 318 recouvre la face interne de l'arceau 31.1. L'arceau 31.1 est le support sur lequel sont regroupés l'ensemble des éléments permettant au système multimédia 31 du système de support et de contrôle 30 de fonctionner.

Le système multimédia comporte un dispositif de communication 310 permettant la transmission et la réception de données et un système d'acquisition et de transmission audio 311 ainsi qu'un système d'acquisition et de transmission vidéo 312.

Le système d'acquisition et de transmission audio 311 comporte un transducteur phonique 314 et un laryngophone 315 intégrés à l'arceau 31.1. Le transducteur phonique 314 forme un haut-parleur transmet à l'opérateur des informations énoncées par un utilisateur distant ou des informations énoncées par le système de contrôle 32 sans que ces instructions orales ne soient altérées. Le laryngophone 315capte les informations énoncées par l'opérateur. Lorsque l'opérateur porte le collier 31, le laryngophone 315 est placé à l'avant du cou au niveau du larynx de l'opérateur afin de capter les vibrations de ses cordes vocales. Dans le mode de réalisation décrit aux [fig.1] et [fig.2], le transducteur phonique 314 est disposé sur l'arceau 31.1 à proximité d'un os de la mâchoire de l'opérateur. Dans le mode de réalisation décrit à la [fig.2], le laryngophone est remplacé par un micro installé dans une paire de lunettes 31.2 portées par l'utilisateur.

Le système d'acquisition et de transmission vidéo 312 comporte une caméra permettant l'enregistrement d'images en continu pendant le fonctionnement du système de support et de contrôle 30. Dans le mode de réalisation décrit à la [fig.2] la caméra est positionnée sur les lunettes 31.2 portées par l'utilisateur, lesdites lunettes 31.2 étant ici comprises dans le système multimédia 31. La caméra 312 définit un champ 3120 de caméra 312 correspondant à la portion d'espace enregistrée, représenté en [fig.4].

Le dispositif de communication 310 comporte une antenne 3100 intégrée dans l'arceau 31.1 du collier 31., pour la transmission à l'utilisateur distant des données du système d'acquisition et de transmission audio 311 et du système d'acquisition et de transmission vidéo 312.

Le collier 31 comporte un bouton de mise sous tension 319 contrôlant la mise sous tension et l'extinction d'une batterie 3190 intégrée à l'arceau 31.1 et alimentant l'ensemble des composants électroniques du collier 31. La mise sous tension de la batterie 3190 et du collier 31 est indiqués par un voyant de fonctionnement 3191. Le voyant de fonctionnement et la batterie 3190 sont disposés sur le collier 31, sensiblement à l'arrière du cou de l'utilisateur, à l'opposé du laryngophone 315.

Aux [fig.1] et [fig.2] l'arceau 31.1 comporte dans le logement dans lequel est disposé la batterie 3190 une unité de contrôle 34 contrôlant l'ensemble des composants électroniques du collier 31 ainsi qu'une carte électronique 316 comportant une pluralité de capteurs. La carte électronique 316 comporte un calculateur 317. Les capteurs 316 permettent chacun de mesurer un paramètre de l'environnement ou un paramètre physiologique de l'opérateur. La carte électronique 316 comporte un capteur de pression, un capteur d'hygrométrie, un capteur de température, un capteur du niveau de CO₂ et de O₂, un altimètre, un radiamètre ou un contaminamètre, ou encore un accéléromètre. Le calculateur 317 traite les données perçues par la pluralité de capteurs pour les transmettre ensuite à l'unité de contrôle 34.

Le collier 31 comporte un système de localisation 319 de l'opérateur permettant à l'utilisateur distant de suivre les déplacements de l'opérateur. Le système de localisation 319 comporte d'une part une puce GPS (de l'anglais Global Positioning System) et d'autre part un émetteur-récepteur associé à un calculateur apte à mettre en œuvre un procédé de biangulation/triangulation des signaux émis par l'émetteur-récepteur du système de localisation 319 et reçus par des émetteurs installés dans le lieu de la mission.

Le système de contrôle 32 comprend une mémoire 320 dans laquelle est stockée au moins une séquence d'instructions audio et/ou vidéo prédéterminées, un moyen de traitement 321 des signaux audio et/ou vidéo et un interface de saisie 322.

Le système de contrôle 32 permet de contrôler le déroulement de la mission de l'opérateur selon un procédé particulier décrit schématiquement, selon un mode de réalisation de l'invention, en [fig.3].

Au début de la mission, lorsque l'opérateur met sous tension, dans une étape 40, le système de support et de contrôle 30, l'unité de contrôle met en oeuvre un test de vérification du système d'acquisition et de transmission audio 311 et/ou vidéo 312, du dispositif de communication 310 et du système de localisation 313. Quand le système de contrôle 32 valide le test, celui-ci est rendu opérationnel et l'opérateur, par l'intermédiaire de l'interface de saisie 322, sélectionne, dans une étape 41, une séquence d'instructions audio et/ou vidéo prédéterminées et enregistrées dans la mémoire 320 du système de contrôle 32. La sélection de la séquence d'instructions entraine une réponse du système de contrôle 32 au cours de laquelle ledit système de contrôle transmet, dans une étape 42, la première instruction de la séquence prédéterminée au dispositif de communication 310 du système multimédia 31. Le dispositif de communication 310 est à son tour agencé pour relayer ladite instruction aux systèmes acquisition audio et/ou vidéo 311, 312. L'instruction transmise dans l'étape 42 décrit une étape de la mission en identifiant au moins un paramètre nécessaire à la réalisation de ladite étape par exemple « tourner la poignée de la vanne 35 ». Le signal audio 311 et/ou vidéo 312 peut être généré par l'utilisateur distant ou de manière automatique par synthèse vocale d'une instruction écrite. Lors de l'exécution de l'instruction par l'opérateur, l'opérateur énonce oralement, dans une étape 43, le geste qu'il est en train de réaliser, par exemple « je tourne la poignée de la vanne 35 » et la caméra 312 du système d'acquisition et de transmission vidéo 312 filme l'action, dans une étape 44, de l'opérateur dans le champ 3120 de ladite caméra 312 tel que représenté en [fig.4]. Les signaux audio 311 et vidéo 312 acquis sont transmis, dans une étape 45, au système de contrôle 32 en réponse à l'instruction de la séquence prédéterminée transmise précédemment. Les mots énoncés par l'opérateur par exemple « je tourne la poignée de la vanne 35 » sont analysés par le système de contrôle et traduits en texte écrit par les moyens de transcription vocaux du moyen de traitement 321. Les moyens de traitement 321 de signaux audio 311 et/ou vidéo 312 du système de contrôle 32 traitent, dans une étape 46, les signaux reçus. Par exemple, le texte transcrit à partir de l'énoncé oral de l'opérateur est comparé à un script prédéterminé. Par exemple encore, les images filmées dans le champs 3120 de la caméra 312 sont également analysés. Dans l'exemple cité, il est demandé à l'opérateur de tourner une poignée de vanne 35, le système de contrôle 32 a enregistré dans la mémoire 320 une image 350 d'une poignée de vanne 35, représentée en pointillés sur la [fig.4] et compare ladite image 350 avec la forme filmée dans le champ 318 de la caméra 312. La comparaison des signaux audio et/ou vidéo reçus par le système de contrôle et comparés avec des données d'un script prédéterminé permet au système de contrôle 32 de valider ou d'invalider l'étape 47. Deux cas se présentent au système de contrôle : le premier cas est celui dans lequel l'étape validée 47 n'est pas la dernière et d'autres étapes sont nécessaires à la réalisation de la mission. Dans ce cas, le système de contrôle 32 relance, dans une étape 470, les étapes précédentes de l'envoi d'une instruction à l'opérateur à la validation de l'étape par le système de contrôle 32, jusqu'à épuisement des instructions de la séquence d'instructions. Le deuxième cas est celui dans lequel l'étape validée 47 est la dernière étape 471 de la mission, le système de contrôle 32 clôt 48 la mission.

Dans un autre exemple de réalisation, l'instruction transmise est « utilise le tournevis cruciforme pour ouvrir le coffre », l'opérateur énonce oralement « j'utilise le tournevis cruciforme pour ouvrir le coffre », le tournevis cruciforme est équipé d'un code barre lu par la caméra, permettant de valider visuellement l'étape.

La description qui précède explique clairement comment l'invention permet d'atteindre les objectifs qu'elle s'est fixée, à savoir proposer un système ergonomique, autonome en énergie pour permettre l'exécution d'opérations d'intervention, dont la communication avec l'encadrant est continue et dont le positionnement sur l'intervenant lui permette d'être libre de ses mouvements sans le gêner dans l'exécution de ses tâches tout en permettant de suivre physiquement et physiologiquement l'état de l'intervenant, en proposant un système de support et contrôle de missions d'intervention techniques, comprenant un système multimédia, destiné à équiper un opérateur un système de contrôle centralisé et distant du système multimédia.

En tout état de cause, l'invention ne saurait se limiter aux modes de réalisation spécifiquement décrits dans ce document, et s'étend en particulier à tous moyens équivalents et à toute combinaison techniquement opérante de ces moyens.

## Revendications

1. Système de support et contrôle (30) de missions d'intervention techniques, comprenant :
a. Un système multimédia (31), destiné à équiper un opérateur, et comportant :
i. Un dispositif de communication (310) pour la transmission et la réception de données,
ii. Un système d'acquisition et de transmission audio (311) ;
iii. Un système d'acquisition et de transmission vidéo (312) ;
b. Un système de contrôle (32) centralisé et distant du système multimédia (31), comprenant une mémoire (320) dans laquelle est stockée au moins une séquence d'instructions audio et/ou vidéo prédéterminées et des moyens de traitement (321) de signaux audio et/ou vidéo ;
**caractérisé en ce que** le système de contrôle (32) est agencé pour :
i. transmettre (42) de façon séquentielle chacune des instructions de la séquence d'instructions au dispositif de communication (310) du système multimédia (31), lequel est agencé pour relayer cette instruction au système d'acquisition et de transmission audio (311) et/ou vidéo (312) pour être transmise à l'opérateur ;
ii. recevoir (45) du dispositif de communication (310) du système multimédia (31), en réponse à chaque transmission (42) d'une instruction, un signal audio (43) et/ou vidéo (44) acquis par le système d'acquisition et de transmission audio (311) et/ou vidéo (312) du système multimédia (31) ;
iii. traiter (46) le signal audio (43) et/ou vidéo (44) reçu par les moyens de traitement (321) de signaux audio et/ou vidéo et valider (47), en fonction de ce traitement (46), l'exécution par l'opérateur de chaque instruction transmise (42), la transmission d'au moins une instruction (42) de la séquence d'instructions étant conditionnée à la validation de l'exécution (47) de l'instruction précédente de la séquence d'instructions.

2. Système (30) selon la revendication précédente, dans lequel les moyens de traitement (321) de signaux audio (43) et/ou vidéo (44) du système de contrôle (32) comprennent un moyen de transcription vocale apte à transcrire un signal vocal (43) en texte, et dans lequel le système de contrôle (32) est agencé, lors de la réception du dispositif de communication (310) de l'équipement multimédia (31) d'un signal vocal (43) acquis par le système d'acquisition et de transmission audio (311), pour transcrire ce signal vocal (43) en texte et stocker ce texte dans ladite mémoire (320) du système de contrôle (32).

3. Système selon l'une des revendications précédentes, dans lequel une pluralité de séquence d'instructions (42) audio et/ou vidéo prédéterminées sont stockées dans la mémoire (320) du système de contrôle (32) en étant chacune associée à un identifiant donné d'une intervention technique à exécuter par un opérateur, et dans lequel le système de contrôle (32) comporte une interface de sélection (322) d'un identifiant d'une intervention technique à exécuter (41) par un opérateur parmi une pluralité d'identifiants.

4. Système (30) selon l'une des revendications précédentes, dans lequel le système de contrôle (30) comporte une interface (322) de saisie d'une séquence d'instructions (42) audio et/ou vidéo prédéterminées, associée à une intervention donnée, chaque instruction (42) décrivant une étape de ladite intervention en identifiant au moins l'un ou plusieurs des paramètres d'étapes suivants : au moins un intervenant à l'étape, au moins une localisation de l'étape, au moins un outil nécessaire l'étape, au moins une pièce à manipuler dans l'étape, au moins un mode opératoire de l'étape.

5. Système (30) selon l'une des revendications précédentes, dans lequel le système multimédia (31) comporte au moins un capteur (316) apte à mesurer un paramètre de l'environnement de l'opérateur ou un paramètre physiologique de l'opérateur, dans lequel le dispositif de communication (310) est agencé pour transmettre au système de contrôle (32) des données relatives audit paramètre mesuré par le capteur (316), et dans lequel le système de contrôle (32) est agencé pour comparer lesdites données reçues à une valeur seuil prédéterminée et pour, en fonction de cette comparaison, émettre un signal d'alerte audio et/ou vidéo au dispositif de communication (310) du système multimédia (31), lequel est agencé pour relayer ce signal d'alerte au système d'acquisition et de transmission audio (311) et/ou vidéo (312) pour être transmis à l'opérateur.

6. Système (30) selon l'une des revendications précédentes, dans lequel le système multimédia (31) comporte au moins un système de localisation (313) de l'opérateur, dans lequel le dispositif de communication (310) est agencé pour transmettre au système de contrôle (32), des données relatives à la position de l'opérateur estimé par le système de localisation (313).

7. Système (30) selon l'une des revendications précédentes, dans lequel le système de contrôle (32) est apte à identifier un début d'une intervention technique à exécuter par un opérateur et à identifier (46) au moins un outil nécessaire à l'exécution de cette intervention, le système de contrôle (32) étant agencé, au préalable de la transmission (42) de la première instruction de la séquence d'instructions associée à cette intervention, pour recevoir du dispositif de communication (310) de l'équipement multimédia (31), un signal vidéo (44) acquis par le système d'acquisition et de transmission vidéo (312) du système multimédia (31) et pour contrôler (46) la présence sur ce signal vidéo, à l'aide du moyen de traitement (321) de signaux audio (43) et/ou vidéo (44), de chaque outil (34) nécessaire préalablement identifié.

8. Système (30) selon l'une des revendications précédentes, dans lequel, pour au moins un signal audio (43) et/ou vidéo (44) reçu du dispositif de communication (310) de l'équipement multimédia (31) en réponse à la transmission (42) d'une instruction, le système de contrôle (32) est agencé pour :
a. Si l'instruction (42) transmise requiert l'utilisation d'un outil (34) donné, contrôler (46) la mention dans le signal audio (43) reçu ou la présence sur le signal vidéo (44) reçu, à l'aide du moyen de traitement (321) de signaux audio et/ou vidéo, de cet outil (34) donné ; et/ou
b. Si l'instruction (42) transmise requiert la manipulation d'une pièce (34) donnée, contrôler (46) la mention dans le signal audio (43) reçu ou la présence sur le signal vidéo (44) reçu, à l'aide du moyen de traitement (321) de signaux audio (43) et/ ou vidéo (44), de cette pièce (34) donnée ; et/ou
c. Si l'instruction (42) transmise requiert un mode opératoire donné, contrôler (46) la mention dans le signal audio (43) reçu ou la réalisation sur le signal vidéo (44) reçu, à l'aide du moyen de traitement (321) de signaux audio et/ou vidéo, de ce mode opératoire donné.

9. Système (30) selon l'une des revendications précédentes, dans lequel l'équipement multimédia (31) est un collier (31) autonome, destiné à être porté par l'opérateur, et intégrant le dispositif de communication (310) et les systèmes d'acquisition et de transmission audio (311) et vidéo (312).

10. Procédé de support et contrôle de missions d'intervention techniques, **caractérisé en ce qu'**il est mis en œuvre par un système de support et contrôle (30) de missions d'intervention techniques selon l'une des revendications précédentes.
